# EUROPEAN PATENT APPLICATION

(11) **EP 2 573 799 A1**
(43) Date of publication of application: **27.03.2013**
(21) Application number: 11182204.5
(22) Date of filing: 21.09.2011
(51) Int. Cl.: H01J 61/44

(54) **Apparatus for promoting D-vitamin production in a living organism**

(71) Applicant: SRLight ApS, 7400 Herning (DK)
(72) Inventor: SRLight ApS, 7400 Herning (DK)
(74) Representative: Høiberg A/S

(57) **Abstract**

The invention regards an apparatus for promoting D-vitamin production in a living organism, comprising at least one lamp assembly, said at least one lamp assembly is adapted to emit polychromatic light, wherein the polychromatic light at least emulates natural light and UV light at wavelengths between 270 nm and 315 nm.

## Description

### Field of invention

The invention relates to an apparatus for promoting D-vitamin production in a living organism, comprising at least one lamp.

### Background of the Invention

It is known in the art that natural light promotes the production of D-vitamins in the human body.

WO 2009/094100 discloses an enhanced UV-emitting fluorescent lamp that provides a UV spectral emission for simultaneously tanning of the human skin and promotion of vitamin D production in the human body. The disclosed lamp is a monochromatic low pressure lamp. Low pressure lamps only supply monochromatic light (just one dominant spectral line). In addition the power rating does not go beyond a few hundreds watt which is a disadvantage if the lamps are to be installed, for example, in an cowhouse at a distance of 3 to 4 meters from the cows.

WO2010/102039 discloses a method for increasing the vitamin D content of a mushroom by exposing the mushroom to a lamp that emits UVA and UVB, but not UVC radiation. It is noted that the mushrooms are only exposed to UVA and UVB radiation but not natural light. In addition, it is noted that there is an increase in the content of D2 vitamin and for example not of the other important D vitamins, such as the D3 vitamin.

### Summary of the invention

Considering the prior art described above, it is an object of the present invention to provide an apparatus and method for promoting D-vitamin production in a living organism in an efficient, cost effective and reliable way.

The object can be achieved by means of 1 an apparatus comprising at least one lamp assembly, said at least one lamp assembly is adapted to emit polychromatic light, wherein the polychromatic light at least emulates natural light and UV light at wavelengths between 270 nm and 315 nm.

By providing a combination of natural light and UV light at wavelengths between 270 nm and 315 nm, the production of D-vitamin in living organisms is enhanced, there is the further advantage that in milk producing mammals, such as cows, the D vitamin content of the milk is increased. Especially, it has been found that the vitamin D3 content is increased, and especially that the vitamin D content in the milk from cows is increased when exposed to polychromatic light comprising both natural light and light in at wavelengths between 270 nm and 315 nm.

Natural light is to be understood as sunlight as it appears on the earth surface during day within the normal variations in intensity and spectrum. The spectrum and intensity of sunlight varies depending on the atmosphere, ozone layer and the position of the sun, and here natural light refer to any sunlight within those normal variations.

Polychromatic light is to be understood as light exhibiting a plurality of colours, in other words that the light comprises a plurality of wavelengths.

In an embodiment, the polychromatic light appears to the naked eye to be continuous, such as polychromatic light at a frequency over 50Hz. The use of a flashing or pulsing light will stress the living organism. Therefore, it is an advantage to ensure that the light appears to be continuous to the living organism, such as an animal. This can be ensured by having a frequency above 50Hz, 100Hz, 200Hz, 500Hz, 1000Hz, 2000Hz or 3000Hz.

Preferably, the apparatus comprises means for removing light of a wavelength under 270 nm. There is health risks involved with exposure to light at a wavelength under 270nm. Therefore, living organisms, such as mammals e.g. cows, can be protected by removing the harming light. The means for removing light with a wavelength under 270nm can be a filter that is transparent to light of a wavelength over 270 and opaque for wavelength under 270nm.

Advantageously, said at least one lamp assembly comprises at least one medium pressure lamp and/or high pressure lamp. When using a medium pressure lamp and/or high pressure lamp, it is possible to reach higher power compared to low pressure lamps. This is especially advantageously when installing the apparatus in a stable, cowhouse or pigsty or any other larger building where the apparatus supplies light to more than one animal. In that case, the apparatus can be positioned at a distance of 2, 3 or 4 or more meters away from the animals, and in order to supply sufficient light to the animals, a high power is preferred. A medium pressure lamp is to be understood as a lamp with a gas pressure of 0.01 to 1 MPa. A high pressure lamp is to be understood as a lamp with a gas pressure of 3 to 10 MPa. A low pressure lamp is to be understood as a lamp with a gas pressure of 0.01 to 133 Pa.

In an embodiment, said at least one lamp assembly comprises a first lamp emulating natural light and a second lamp providing UV light at wavelengths between 270 nm and 315 nm. In this embodiment, the first lamp can be any lamp that emulates natural light, such as a sulphur lamp, and the second lamp can be a fluorescent lamp or a medium pressure lamp, preferably a medium pressure lamp with enhanced energies at wavelength between 270nm and 315nm. There can be any number of lamps in order to secure that the area covered by the emitted light is as extensive as possible.

In an embodiment, said at least one lamp assembly comprises a doped lamp, preferably a doped sulphur lamp, a mercury lamp and/or a xenon lamp, which emits natural light in conjunction with UV light. In this embodiment, it is possible to use only one lamp as it can provide the full spectrum needed.

Advantageously, the doped lamp is a sulphur lamp, a mercury lamp and/or a xenon lamp, doped with a metal, non-metal or metalloids, preferably tin, lead, iron, phosphorus, sulphur, selenium, boron, silicon, germanium, arsenic, antimony and /or tellurium. Doping the lamp with metal and especially with the mentioned metals will enhance the intensities of the UV light at wavelengths between 270 nm and 315 nm.

A further aspect of the present invention is the use of an apparatus as described herein to increase D-vitamin production in animals, preferably cows. The apparatus as described above can be used to enhance the D-vitamin production within the living organism of an animal. Thus, the D-vitamin content in the meat form the animals exposed to the apparatus described above. This can prevent humans eating the meat from suffering from vitamin D deficiency. These animals can be birds, such as chickens and/or mammals, such as cows, pigs, goats and/or lambs.

The invention also regards the use of an apparatus as described to increase D-vitamin content in milk from a mammal, preferably a cow. Increasing the vitamin D content in milk by using of the present invention has the advantage that humans consuming the milk can prevent vitamin D deficiency. In parts of the earth away from equator, the daytime light can be limited during winter. This is, for example, the case in northern Europe during the months November to March. During that time, the lack of exposure to the sun can cause people to suffer from vitamin D deficiency. In any case the sun is only important if the cows are outside. In Northern Europe, and other industrialised countries at the same latitude north or south, the majority of milk cows are inside a cowhouse. In those cases, drinking milk with vitamin D can help prevent the lack of vitamin D in the human body.

An additional aspect of the present invention is a cowhouse comprising at least one apparatus according to the present invention. As the apparatus emits both visible light and UV light, a cowhouse with the apparatus of the present invention does not need any additional lightening system. The present invention can substitute the conventional illumination of the cowhouse which saves money at installation as only one illumination system is needed.

In addition, the invention regards a method for increasing D-vitamin content in milk, preferably cow milk, by exposing an animal, preferably cow, producing said milk to polychromatic light, wherein the polychromatic light at least emulates natural light and UV light at wavelengths between 270 nm and 315 nm. By using the method, it is possible to increase vitamin D content in milk which is beneficial to the milk consumer. Lack of vitamin D can lead to health problems, such as impaired bone mineralization, and bone softening diseases. Those deficiencies can be avoided if milk with increased vitamin D content is consumed.

Advantageously, the polychromatic light appears to the naked eye to be continuous, such as polychromatic light with a frequency over 50Hz. In order to avoid stressing the animals, the light should appear to be continuous to the animal. A stressed animal can result in a decreased milk production and is therefore not desirable. The frequency can for example be above 50Hz, 100Hz, 200Hz, 500Hz, 1000Hz, 2000Hz or 3000Hz.

Preferably, the animal is only exposed to light at a wavelength over 270 nm. Light in the UV range can be harmful to living organisms. Therefore, it is desirable to limit the exposure of the animals to light at a wavelength under 270. This can for example be achieved by using a filter which remove light under the desired wavelength and/or designing the light emitter in such a way that no light under 270nm is produced.

In an embodiment, polychromatic light is provided by a plurality of lamps. This can make the installation easier and in addition it is possible to turn off only some of the lamps. This can be advantageous, if for example a first lamp for natural light and a second lamp for the light at wavelengths between 270 nm and 315 nm are used. Then the light having wavelengths between 270 nm and 315 nm can be turned on for only a limited time for example 2 to 3 hours every day and the natural light can be on the entire day and be used as the primary illumination of the building.

### Description of the drawings

The invention will in the following be described in greater detail with reference to the accompanying drawings:
- Fig. 1: a graph of a spectrum for a doped mercury UV lamp.
- Fig. 2: a graph of a spectrum for sulphur lamps.
- Fig. 3: a schematic view of lamp according to the present invention.
- Fig. 4: a second schematic view of lamp according to the present invention.
- Fig. 5: a graph showing test results.

### Detailed description of the invention

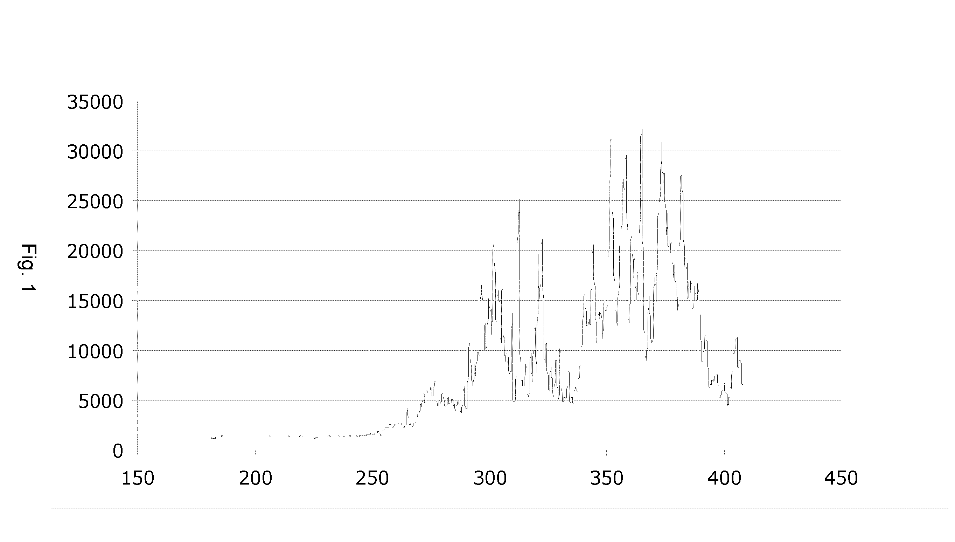

Fig 1 shows intensities as a function of wavelength of a doped mercury UV lamp. As can be seen, there is light in the range from approximately 250 nm and up. A line is shown at 270 nm because a filter can be installed in order to remove light under that wavelength.

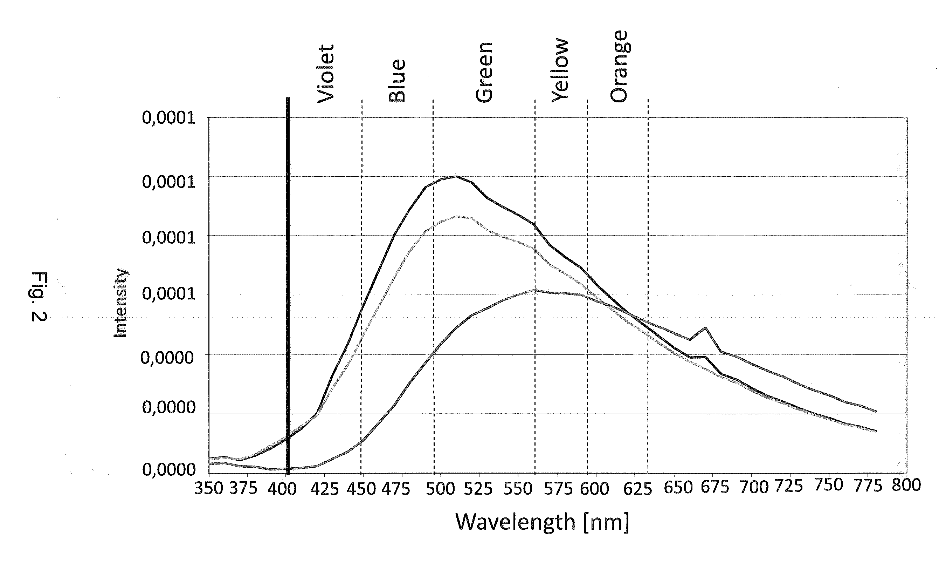

Fig. 2 shows a graph for sulphur lamps of different powers. The spectrum is very similar to daytime solar light at the earth's surface. Therefore, a sulphur lamp can be used to provide natural light. Sulphur lamps are known in the art and will not be described in detail. As can be seen, there is little UV light. UV light is electromagnetic radiation with a wavelength of between 10nm and 400nm. A sulphur lamp can be doped with metals in order to achieve light emission as close to natural light as technically possible.

A lamp assembly according to the present invention can be a combination of the doped UV lamp and a mercury lamp, as an alternative the sulphur lamp described above can be used. If the lamp assembly comprises these two lamps, it is possible to regulate the UV and the natural light independently. As an example, such a lamp assembly can be installed in a cowhouse, then the mecury lamp can be used as illumination to represent the daytime and so that the farmer can work in the cowhouse. The UV lamp can then be turned on and off for only a limited amount of time each day, for example 1, 2 or 4 hours every day. By using the natural light lamp (here exemplified as a mecury lamp) as illumination in the cowhouse no other lighting installation is needed. Hence, the costs of the normal illumination can be reduced.

In another embodiment the lamp assembly comprises one doped mercury lamp. The lamp can be doped with, for example Iron, tin,lead, phosphorus, sulphur, selenium, boron, silicon, germanium, arsenic, antimony and /or tellurium.. Such a lamp both emulates natural light and has enhanced intensities in the UV area. The light with wavelength under 270 nm can then removed by use of a filter. When using such a lamp there is used only one lamp needs to be installed as it can be used as normal illumination. In addition it will enhance the vitamin D production in living organisms exposed to its light. This means that a framer only needs to install this type of lamps in a cowhouse in order to increase the vitamin D content of the produced milk.

The following table includes examples of different lamp types that can be used in a lamp assembly.

| **Lamp type** | **no.** | **Min. nm** | **Max. nm** |
|---|---|---|---|
| Mercury florescent low pressure | 1 | 270 | 315 |
| Mercury medium pressure doped | 2 | 270 | 315 |
| Mercury medium pressure doped | 3 | 270 | 900 |
| Mercury medium pressure | 4 | 380 | 900 |
| Mercury high pressure | 5 | 260 | 900 |
| Xenon high pressure | 6 | 300 | 900 |
| Mercury high pressure doped | 7 | 270 | 900 |
| Xenon high pressure doped | 8 | 270 | 900 |
| Sulphur | 9 | 380 | 900 |
| Plasma and/or incandescent lamp. (Solar Simulator) | 10 | 380 | 900 |
| Doped Plasma and/or doped incandescent lamp; (Solar Simulator) | 11 | 270 | 900 |

With use of examples of the lamps in the table different embodiments of a lamp assembly can be made.

The following table has examples of lamp assemblies made from the lamp types from the above shown table.

| **Lamp and combinations of lamps** | **no.** | **no.** |
|---|---|---|
| Lamp assembly A | 1 | 4 |
| Lamp assembly B | 1 | 5 |
| Lamp assembly C | 1 | 6 |
| Lamp assembly D | 1 | 9 |
| Lamp assembly E | 1 | 10 |
| Lamp assembly F | 2 | 4 |
| Lamp assembly G | 2 | 5 |
| Lamp assembly H | 2 | 6 |
| Lamp assembly I | 2 | 9 |
| Lamp assembly J | 2 | 10 |
| Lamp assembly K | 3 | |
| Lamp assembly L | 5 | |
| Lamp assembly M | 7 | |
| Lamp assembly N | 8 | |
| Lamp assembly O | 11 | |

Thus, the lamp assemblies A to J are a combination of two lamp types and the lamp assemblies K to O only use one lamp.

As the lamp assembly is to be used as lighting, it is preferred that the lighting appears to be continuous. A pulsed lamp will not only stress the animals but also make it difficult for the farmer to work, most of the known UV and natural light lamps are to some extend pulsed but as long as the pulses are higher than 50Hz it will appear to both animals and humans to be continuous.

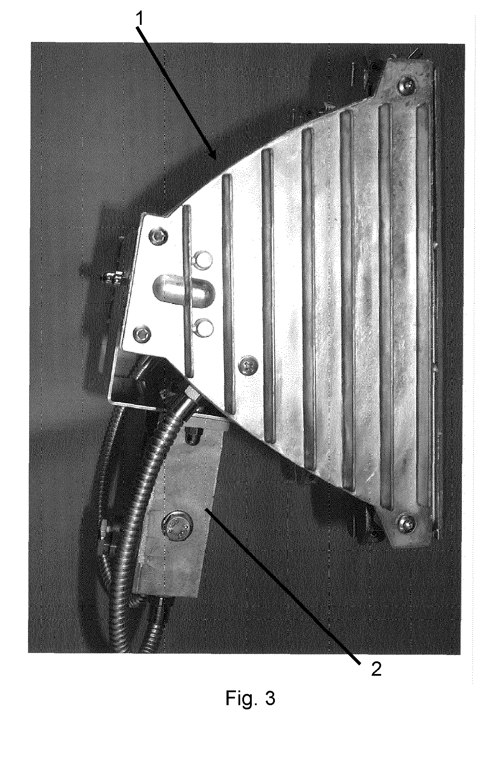

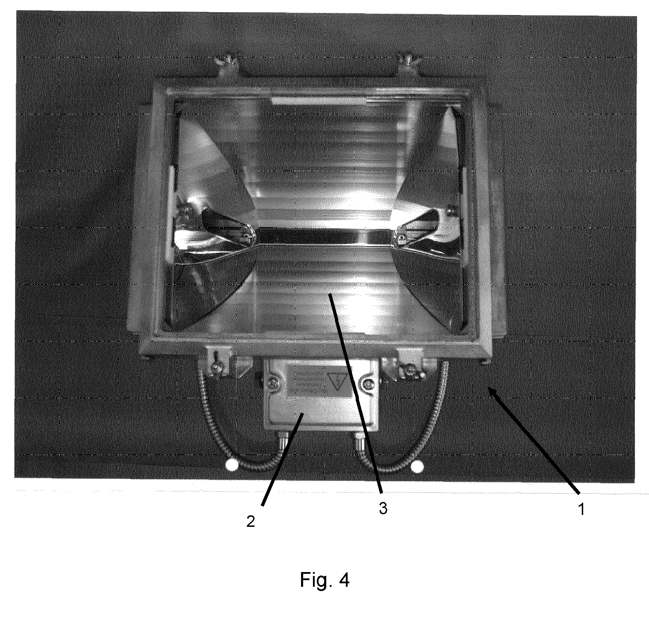

The figures 3 and 4 shows a lamp house 1 that can be used for the UV lamp as described above, it has an electronic control box 2 and a reflector 3.

In an embodiment, the lamp assembly has a lamp that can provide both natural light and UV light at the same time. Such a lamp can be a mercury, sulphur, xenon lamp doped with metal, non-metals or metalloids, for example, any combination of tin (Sn), lead (Pb), iron (Fe), phosphorus (P), sulphur (S), selenium (Se), boron (B), silicon (Si), germanium (Ge), arsenic (As), antimony (Sb) and /or tellurium (Te).. These elements can enhance the intensities in the 270nm to 315 nm range.

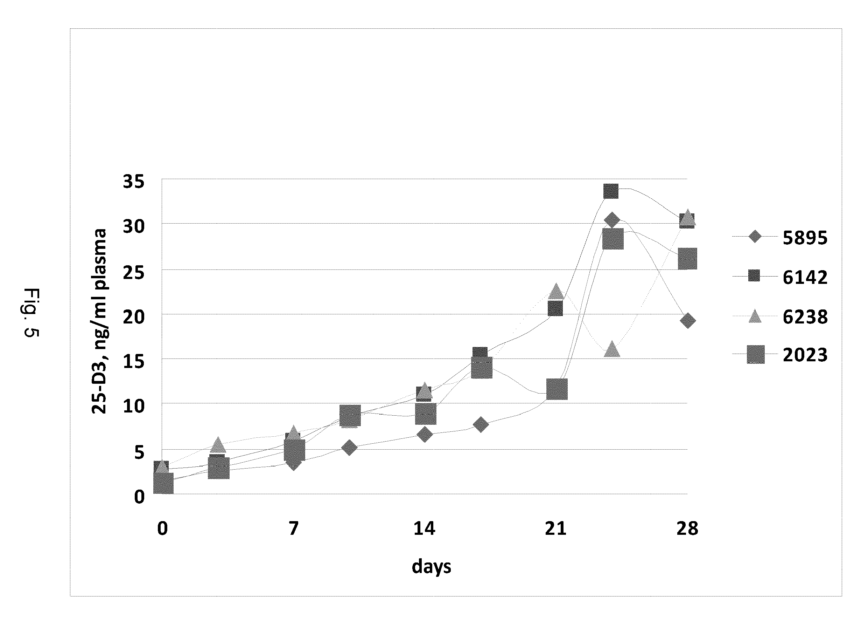

A test of an embodiment of the invention has been performed. Here, the UV lamp having the spectrum as shown in fig. 1 was used together with a sulphur lamp to illuminate cows. The lamp assembly was installed 3 to 3.5 meters from the cows in the test. The lamp was turned on for 30 minutes every 24 hours. This was repeated for 28 days. The test was performed on four cows, having the numbers: 5895, 6142, 6238 and 2023. The cows were milked every day and the vitamin D3 content of the milk was measured for each of the four cows. The result of the test is shown in fig. 5. It can be seen that the content of D3 vitamin increases from about 3 ng/ml to about 25 ng/ml. This is a substantial increase in the D vitamin content of the milk.

## Claims

1. Apparatus for promoting D-vitamin production in a living organism, comprising at least one lamp assembly, said at least one lamp assembly is adapted to emit polychromatic light, wherein the polychromatic light at least emulates natural light and UV light at wavelengths between 270 nm and 315 nm.

2. Apparatus according to claim 1, wherein the polychromatic light appears to the naked eye to be continuous, such as polychromatic light at a frequency over 50Hz.

3. Apparatus according to any of the preceding claims, wherein the apparatus comprises means for removing light of a wavelength under 270 nm.

4. Apparatus according to any of the preceding claims, wherein said at least one lamp assembly comprises at least one medium pressure lamp and/or high pressure lamp.

5. Apparatus according to any of the preceding claims, wherein said at least one lamp assembly comprises a first lamp emulating natural light and a second lamp providing UV light at wavelengths between 270 nm and 315 nm.

6. Apparatus according to any of the preceding claims, wherein said at least one lamp assembly comprises a doped lamp, preferably a doped sulphur lamp, a mercury lamp and/or a xenon lamp, which emits natural light in conjunction with UV light.

7. Apparatus according to claim 6, wherein the doped lamp is a sulphur lamp, a mercury lamp and/or a xenon lamp, doped with a metal, non-metal or metalloids, preferably tin, lead, iron, phosphorus, sulphur, selenium, boron, silicon, germanium, arsenic, antimony and /or tellurium.

8. Use of apparatus according to any of the preceding claims to increase D-vitamin production in animals, preferably cows.

9. Use of apparatus according to any of the claims 1 to 7 to increase D-vitamin content in milk from a mammal, preferably a cow.

10. A cowhouse comprising at least one apparatus according to any of the claims 1 to 7.

11. Method for increasing D-vitamin content in milk, preferably cow milk, by exposing an animal, preferably cow, producing said milk to polychromatic light, wherein the polychromatic light at least emulates natural light and UV light at wavelengths between 270 nm and 315 nm.

12. Method according to claim 11 wherein the polychromatic light appears to the naked eye to be continuous, such as polychromatic light with a frequency over 50Hz.

13. Method according to any of the claims 11 to 12, wherein the animal is only exposed to light at a wavelength over 270 nm.

14. Method according to any of the claims 11 to 13, polychromatic light is provided by a plurality of lamps.
